# EUROPÄISCHE PATENTSCHRIFT

(11) **EP 1 282 381 B1**
(45) Veröffentlichungstag und Bekanntmachung des Hinweises auf die Patenterteilung: **25.06.2008**
(21) Anmeldenummer: 01960227.5
(22) Anmeldetag: 18.05.2001
(51) Int. Cl.: A61B 5/03

(54) **VORRICHTUNG ZUM BESTIMMEN EINES EICH- UND/ODER ABSOLUTWERTES EINES INTRAKRANIELLEN DRUCKES**
DEVICE FOR DETERMINING A CALIBRATION AND/OR ABSOLUTE VALUE OF AN INTRA-CRANIAL PRESSURE
DISPOSITIF POUR DETERMINER UNE VALEUR ETALON OU ABSOLUE D'UNE PRESSION INTRACRANIENNE

(30) Priorität: 19.05.2000 DE 20008923 U
(43) Veröffentlichungstag der Anmeldung: 12.02.2003
(73) Patentinhaber: Compumedics Germany GmbH, 78224 Singen (DE)
(72) Erfinder: PAULAT, Klaus, 78354 Sipplingen (DE)
(74) Vertreter: Behrmann, Niels
(86) Internationale Anmeldenummer: PCT/EP2001/005734
(87) Internationale Veröffentlichungsnummer: WO 2001/087148

(56) Entgegenhaltungen:
- EP-A- 0 933 061
- WO-A-80/00913
- DE-A- 19 903 247
- US-A- 4 841 986

## Beschreibung

Die vorliegende Erfindung betrifft eine Vorrichtung zum Bestimmen eines Eich- und/oder Absolutwertes eines intrakraniellen Druckes im Schädel eines Menschen.

Aus der DE 199 03 247 A1 bzw. der EP 0 933 061 A1 der Anmelderin ist eine Vorrichtung zur Messung des intrakraniellen Druckes und/oder der Compliance am Schädel eines Lebewesens bekannt. Diese bekannten, nicht invasiven Vorrichtungen ermöglichen es, einfach und zuverlässig, und insbesondere ohne Belastung des Patienten durch operative Eingriffe, den Schädelinnendruck (intrakranieller Druck ICP) zu messen, wobei insbesondere bei durch Unfälle bedingten traumatischen Zuständen o. ä. eine genaue Kenntnis des ICP sowie dessen zeitlichen Verlauf ein wichtiges Diagnoseinstrument in der Therapie ist.

Während die genannten Schriften zum Stand der Technik, über eine Verlaufsermittlung des ICP hinaus, insbesondere auch eine Bestimmung des Absolutwertes des ICP durch Vergleich bzw. Analyse von Messwerten bzw. Extremwerten entlang der Druckverlaufskurve ermöglichen, so ist jedoch diese Vorgehensweise relativ aufwendig, ungenau und benötigt zunächst einmal einige Verlaufswerte, um einen -- für die Diagnose wichtigen -- Absolutwert des ICP zu ermitteln.

Aufgabe der vorliegenden Erfindung ist es daher, eine Vorrichtung zu schaffen, die eine vereinfachte bzw. verbesserte (insbesondere auch beschleunigte) Ermittlung des Absolutwerts des ICP ermöglicht, und die insbesondere auch im Zusammenhang und Ergänzung mit den eingangs beschriebenen intrakraniellen Druckmessgeräten verwendbar ist.

Die Aufgabe wird durch die Vorrichtung mit den Merkmalen der Ansprüche 1 und 2 gelöst; vorteilhafte Weiterbildungen der Erfindung sind in den Unteransprüchen beschrieben.

Der vorliegenden Erfindung liegt das Prinzip zugrunde, den Öffnungszustand der Brückenvenen im Schädel als Indikator für den Hirndruck zu verwenden: Normalerweise sind die zwischen der Gehirnoberfläche und der Schädelinnenfläche verlaufenden Brückenvenen durch den Hirndruck zusammengedrückt, da der ICP geringfügig (etwa 5 mm Hg) über dem venösen Druck liegt. Insoweit wirken die Brückenvenen als Ventile. Steigt jedoch der venöse Druck in den Brückenvenen über den ICP an, würde dies die Brückenvenen zum kurzzeitigen Erweitern bringen, was einen venösen Volumenzuwachs im Gehirn darstellt, der wiederum mit einem entsprechenden kurzzeitigen ICP-Anstieg verbunden ist.

Die vorliegende Erfindung sieht nunmehr erfindungsgemäß Mittel zum Erzeugen und/oder Beeinflussen des venösen Innendrucks im Schädel vor, mit dem Ziel, diesen ÖffnungszuStand der Brückenvenen herbeizuführen (nämlich, zumindest kurzzeitig, einen minimal höheren Druck als den ICP zu erzeugen, was zu einem Flattern der so entlasteten Brückenvenen führt). Die erfindungsgemäß ferner vorgesehenen Sensoreinheiten sind in der Lage festzustellen, wann die Brückenvenen geöffnet sind (genauer gesagt: denjenigen Zeitpunkt zu ermitteln, zu dem die Brückenvenen bei ansteigendem venösen Druck ventilartig öffnen), und der zu diesem Zeitpunkt geltende Druck entspricht dann dem aktuellen ICP.

Gemäß einer bevorzugten Weiterbildung ist vorgesehen, die Erkennung, wann die Brückenvenen geöffnet sind, mittels eines dem venösen Druck überlagerten modulierten Signals vorzunehmen, wobei dann im Falle geöffneter Brückenvenen diese Modulation in dem durch die Sensoreinheit gemessenen ICP-Signal erkennbar sein muss. Für eine einfache Realisierung der Erfindung reicht es jedoch auch aus, die durch die Atmung erzeugte Signaländerung zu verwenden und mittels der Sensoreinheit zu überprüfen, bei welchem Pegel des venösen Drucks eine durch Atmung bedingte Modulation des ICP-Sensorsignals (mit der Atemfrequenz) erkennbar ist; dieses ist dann der Zeitpunkt bzw. der venöse Druck, bei welchem die Brückenvenen geöffnet sind, mithin also venöser Druck und ICP gleich groß sind.

Während, gemäß einer bevorzugten Ausführungsform, der venöse Innendruck durch einen Gegendruck für das Ausatmen bildende, geeichte bzw. quantifizierbar einstellbare Geräte realisiert werden kann (sogenannter positiver endexpiratorischer Druck PEP), so besteht eine alternative Möglichkeit darin, eine definierte Jugulariskompression zu erzeugen, die etwa mittels einer (einen vorbestimmten, ablesbaren Druck erzeugenden) Blutdruckmanschette, etwa am Hals des Patienten, erzeugt werden kann.

Die Überwachung bzw. Signalerfassung des ICP erfolgt mittels der aus den genannten Schriften zum Stand der Technik bekannten Vorrichtungen, wobei mit Mitteln der digitalen Signalauswertung, der Zeitpunkt der Ventilöffnung durch die Brückenvenen erkannt wird, nämlich auf Grund der druckbedingt deutlichen Signaländerung (so wird im Fall der Nutzung des Atmungssignals erst bei geöffneter Brückenvene eine deutliche, atmungsbedingte Modulation des ICP-Signals erkennbar).

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
- Fig. 1:: ein schematisches Blockschaltbild der Vorrichtung zum Bestimmen eines Eich- oder Absolutwerts eines intrakraniellen Druckes gemäß einer ersten, bevorzugten Ausführungsform der vorliegenden Erfindung;
- Fig. 2:: eine schematische Ansicht der Physiologie im Schädelinneren eines Menschen;
- Fig. 3:: ein Signal-Zeitdiagramm als. Ausgangssignal der erfindungsgemäßen Sensoreinheit mit dem intrakraniellen Druckverlauf bei geschlossenen Brückenvenen, d.h. venösem Druck unterhalb des ICP;
- Fig. 4:: ein Signal-Zeitdiagramm mit einem Druckverlauf als Ausgangssignal der Sensoreinheit bei geöffneten Brückenvenen (d.h. venösem Druck größer oder gerade gleich dem ICP) und
- Fig. 5 bis 7: Schaltbilder und Signaldiagramme der bekannten Ausführungen der Sensoreinheit.

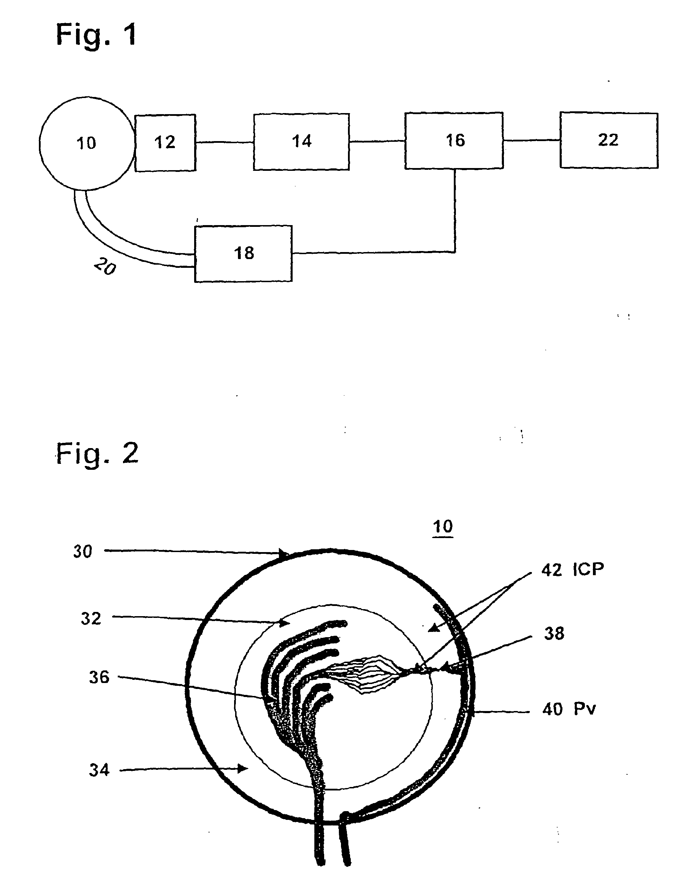

Wie in Fig. 1 gezeigt, ist am menschlichen Schädel (10) eine Sensoreinheit (12) angeschlossen, die in einer der aus der EP 0 933 061 A1 bekannten Weisen in der Lage ist, nicht-invasiv den intrakraniellen Druck im Schädel (10) zu messen, wobei sich zur Realisierung der Sensoreinheit (12) sowohl die Vorgehensweise mittels Ohrstopfen, als auch mittels kapazitiv wirkendem Bewegungsaufnehmer gemäß Offenbarung der genannten Schriften eignet (prinzipiell ist es auch von der vorliegenden Erfindung nicht ausgeschlossen, die erfindungsgemäße Sensoreinheit durch eine invasive intrakranielle Druckmessvorrichtung, etwa mittels ansonsten bekannter, invasiv einzusetzender Drucksonde, zu realisieren).

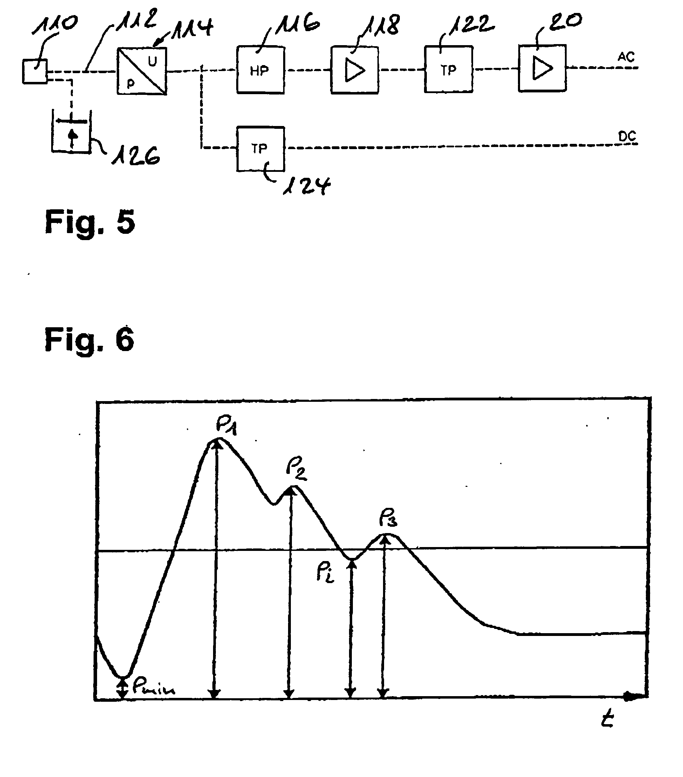

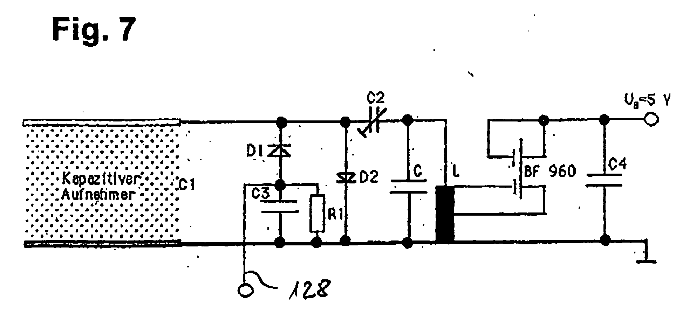

Zunächst wird anhand der Fig. 5 bis 7 die Realisierung der Sensoreinheit 12, einerseits mittels Ohrstopfen (Fig. 5), andererseits mittels Bewegungsaufnehmer (Fig. 7) erläutert:

Gemäss Fig. 5 sind Verschlussmittel in Form eines Ohrstopfens od.dgl. Element so ausgebildet, dass sie luftdicht abschließend in die Gehörgangöffnung eines menschlichen Ohrs eingesetzt werden können. Der Ohrstopfen 110 ist über einen Druckschlauch 112 mit einem extern vorgesehenen Druckaufnehmer 114 versehen, der über den Druckschlauch 112 in Verbindung mit dem vom Ohrstopfen 110 verschlossenen Gehörgang steht.

Der Druckaufnehmer 114 wandelt das gemessene Drucksignal in eine auswertbare, elektrische Spannung um, die zweikanalig zur weiteren Auswertung aufbereitet und dann einer (in der Figur nicht gezeigten) Auswert- und Anzeigeeinheit zugeführt wird. Genauer gesagt wird in einem ersten Zweig der Wechselspannungsanteil mittels eines Hochpasses 116, der eine Grenzfrequenz von beispielsweise 0,5 Hz aufweist, gefiltert, in nachfolgenden Verstärkerstufen 118, 20 um etwa den Faktor 20 bis 50 verstärkt und mittels eines Tiefpasses (TP) 22 mit einer oberen Grenzfrequenz von maximal 30 Hz in seiner Bahnenbreite auch nach oben hin beschränkt. Das aus diesem zweig resultierende AC-Signal steht dann für eine weitergehende Signalaufbereitung, etwa mittels einer Sample-Schaltung (Takt z.B. 200 Hz, Auflösung 12 BIT) zur Verfügung, wobei hierdurch die Grundlage für weitergehenden, rechnerischen Signalauswertung des in Fig. 6 exemplarisch gezeigten Signals und zum Ausgeben entsprechender ICP-Druck-Information für eine Bedienperson einer (nicht gezeigten) Anzeigeeinheit geschaffen wird.

In einem zweiten Auswertezweig wird das Ausgangssignal des Druckaufnehmers 114 mittels eines Tiefpasses 24 einer Grenzfrequenz von beispielsweise 0,5 Hz gefiltert und steht dann unmittelbar als DC-Signal für eine weitere Auswertung zur Verfügung, die, gegenüber einer Herzperiode, auf einen längeren Zeitpunkt gerichtet ist.

Unter Bezug auf Fig. 6 wird nunmehr eine typische, durch den Druckaufnehmer 114 zu erfassende Signalform beschrieben, die in der gezeigten Weise mit nachgeschalteter Signalauswertung das Bestimmen des Gehirndruckes bzw. dessen Verlauf ermöglicht.

Die Fig. 6 verdeutlicht den exemplarischen Verlauf des elektronisch auswertbaren Drucksignals des Druckaufnehmers 114 über eine vom Herzschlag (Herzzyklus) bestimmte Periode eines Menschen.

Es zeigt sich, dass der bezogen auf eine Referenzlinie jeweils gemessene Druckwert P ein anfängliches Minimum Pₘᵢₙ aufweist und daraufhin steil zu einem Maximalwert P₁ ansteigt; im weiteren Kurvenverlauf folgen charakteristische, in ihrer Tendenz absteigende Schwankungen mit nachfolgenden Maxima P₂ und P₃. Ein vor P₃ liegender, minimaler Extremwert P₁ existiert häufig als tiefer Einschnitt (Inzisur) im Kurvenverlauf (lokales Minimum).

Durch die erfindungsgemäße Vorrichtung ist ein solcher charakteristischer Druckkurvenverlauf der signalanalytischen Auswertung zugänglich, wobei insbesondere die absoluten Pegel sowie der maximale Signalhub, nämlich das Verhältnis von P₁ zu Pₘᵢₙ bzw. der Abstand zwischen diesen charakteristisch sind, ebenso wie das Verhältnis von P₁ zu P₂. Insbesondere ist das erste dieser Amplitudenverhältnisse unmittelbar proportional zum intrakraniellen Druck, so dass aus dieser Messung eine unmittelbare Berechnung und Anzeige des Gehirndruckes bzw., bei mehrerern, aufeinanderfolgenden Perioden, dessen Verlaufs, möglich ist. Zusätzlichen Einfluss bzw. eine zusätzliche Möglichkeit zur Bestimmung des Absolutdruckes im Rahmen der Erfindung ergibt sich aus der Analyse eines zeitlichen Abstandes zwischen den aufeinanderfolgenden Maxima P₁, P₂ und P₃.

Gegenüber der Darstellung in Fig. 6, die einen typischen wechselsignalverlauf im ersten Zweig gemäß Fig. 5 beschreibt, ist der Gleichspannungsverlauf in seinen möglichen Schwankungen wesentlich träger und wird üblicherweise über einen mehrminütigen bis gar mehrstündigen Zeitraum überwacht, um insbesondere auch Rückschlüsse auf Langfrist-Veränderungen des Gehirndruckes ziehen zu können.

Schließlich zeigt die Darstellung der Fig. 5 einen schematisch dargestellten Druck- bzw. Volumengeber 26, mit welchem bei eingesetztem Ohrstopfen 110 ein zusätzliches Volumen eines Gases oder eines Fluids in den geschlossenen Gehörgang eingebracht werden kann mit dem Ergebnis, dass auf das Trommelfell am dem Ohrstopfen 110 entgegengesetzten Ende ein zusätzlicher Druck aufgebracht wird, dessen Wirkung auf das Trommelfell, in Form von Anstiegzeit, Reaktionszeit od.dgl. dann wiederum über die in der Fig. 5 dargestellte Messwertkette erfasst und nachfolgend ausgewertet werden kann.

Fig. 7 zeigt zum zweiten, unabhängigen Erfindungskomplex eine typische Realisierungsform einer der auf den Schädel abnehmbar aufsetzbaren Vorrichtung zur Erfassung der Relativbewegung zugeordneten Signalaufbereitung. Genauer gesagt ist der in der Fig. 7 schematisch gezeigte kapazitive Aufnehmer C1 das Bewegungselement, welches so auf dem Schädel befestigt ist, das als Reaktion auf die Relativbewegung zweier Schädelplatten eine kapazitive Änderung von C1 auftritt (C1 besitzt im dargestellten Beispiel eine Kapazität von z.B. 20 pF). Mittels eines einen kapazitiven spannungsteiler aufweisenden Oszillators wird am Signalausgang 28 ein zur weiteren Auswertung geeignetes und vorgesehenes Spannungssignal erzeugt, welches analog der in Fig. 5 dem Druckaufnehmer 114 nachgeschalteten Beschaltung weiter aufbereitet und dann einer rechnergestützten Signalverarbeitung zugeführt werden kann. Der etwa als Wechselspannungssignal erhaltene Signalverlauf entspricht dem in Fig. 6 dargestellten.

Typische Parameter für die Schaltung der Fig. 7 sind: Entladewiderstand R1 240 kohm, Ladekondensator C3 22 nF; Schwingkreis-Kondensator C 150 pF; Trimmkondensator C2 10....30 pF; Siebkondensator C4 82 nF; schwingkreis-Induktivität L 23µH; Gleichrichterdioden D1 D2 z.B. 1N 4148.

Der Sensoreinheit (12) ist, wie in Fig. 1 gezeigt, eine Signalauswerteeinheit (14) nachgeschaltet, die das von der Sensoreinheit (12) ausgegebene Signal analysiert und daraufhin auswertet, ob (und ggf. mit welcher Amplitude) in dem von der Sensoreinheit (12) erfassten Drucksignal ein durch die Atmung bzw. in der Atemfrequenz moduliertes, überlagertes Signal vorliegt. Eine nachgeschaltete Steuereinheit (16) empfängt das Ausgangssignal der Signalauswerteeinheit (14) ; und zusätzlich ein aktuellen venösen Druck anzeigendes Signal von Venendruckmitteln (18), die, schematisch mit einer Leitung (20) gezeigt, einem in die etwa als Schlauch ausgebildete Leitung (20) ausatmenden Menschen einen definierten, bevorzugt einstellbaren, geeichten Gegendruck entgegenbringen, so dass, im gezeigten Ausführungsbeispiel der Fig. 1, mit Hilfe der Atmung eine Beeinflussung des venösen Drucks (Aufgabe und Zweck der Mittel 18) möglich ist.

Eine der Steuereinheit (16) nachgeschaltete Auswerteeinheit (22) ist eingerichtet, einer durch die Sensoreinheit (12) bzw. die Signalauswertung (14) erfassten, einer Öffnung der Brückenvene entsprechenden sprunghaften Signaländerung einen aktuellen Venendruck gemäß Ausgabesignal der Mittel (18) zuzuordnen, so dass als Ergebnis ein ICP-Absolutdruck (der nämlich gleich dem venösen Innendruck zum Zeitpunkt der Brücken-(Ventil-)Öffnung ist), zuzuordnen.

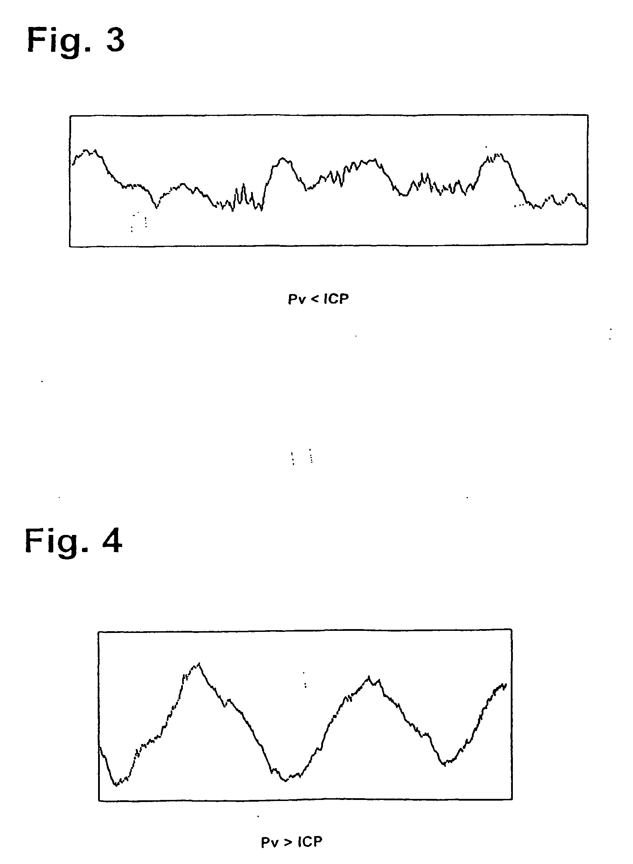

Vor einer Diskussion typischer Signalverläufe anhand der Figuren 3 und 4 sollen zunächst die im Rahmen der vorliegenden Erfindung vorteilhaft ausgenutzten physiologischen Verhältnisse im Schädelinneren erläutert werden: Der schematisch gezeigte Schädelknochen (30) bildet die äußere Begrenzung des Schädels (10); der sog. subdurale Spalt 34 (der Hirnflüssigkeit enthält) bildet den Zwischenraum zwischen dem Gehirn (32) und dem Schädelknochen (30). Das Arteriensystem (36) im Gehirn wird mittels einer schematisch gezeigten Brückenvene (38) mit dem (im subduralen Spalt außenliegenden) Venensystem (40) verbunden, wobei normalerweise der Druck im Venensystem Pv (um typischerweise 5 mm Hg) geringer ist als der Hirninnendruck ICP (42), also die Brückenvene (38) geschlossen ist (über ihr entsprechend ein Druckabfall stattfindet). Gelingt es jedoch, den venösen Innendruck Pv soweit zu erhöhen, dass dieser den Hirndruck (42) übersteigt, so öffnet die Brückenvene (38), und insbesondere Druckschwankungen (Modulationen) des venösen Druckes Pv lassen sich dann als Änderungen des intrakraniellen Druckes ICP abbilden, wie es etwa bei durch Atmung bedingter Änderung von Pv der Fall ist (mit anderen Worten: erst wenn Pv den aktuellen Absolutwert des ICP übersteigt, enthält der gemessene ICP etwa die atmungsbedingte Modulation, so dass der Schwellwert von Pv gleich dem Absolutwert von ICP ist und entsprechend weiteren Messungen und Beobachtungen des ICP zugrunde gelegt werden kann).

Dieser Umstand lässt sich aus einem Vergleich der Figuren 3 und 4 erkennen, die in etwa dieselbe zeitliche Auflösung in horizontaler Richtung besitzen: Während, wie in Fig. 3 gezeigt, bei Pv < ICP in dem gemessenen intrakraniellen Drucksignal praktisch keine klare atmungsbedingte Modulation enthalten ist, so wird diese in Fig. 4 (Pv > ICP) sehr deutlich und auswertbar, insbesondere mit dem Zweck, durch Einheit (14) den Öffnungszustand der Brückenvene (38) zu detektieren.

Die vorliegende Erfindung ist nicht auf die gezeigte Ausführungsform beschränkt; so bietet es sich prinzipiell an, jegliche geeignete (messbare) Vorgehensweise zum

Beeinflussen oder Einstellen des vorbestimmten venösen Innendrucks zu verwenden, um so ein gezieltes, messbares Öffnen der Brückenvenen zu erzwingen. Ein gegenüber der Atmungs-Gegendruckeinheit (18) (Fig. 1), die z.B. mittels eines in eine vorbestimmte Wassersäule hineinreichenden Schlauchs oder dergleichen realisiert sein kann, alternative Möglichkeit besteht in dem Beaufschlagen des Halses des Patienten (mittelbar damit also auch der außenliegenden Vene) mit vorbestimmtem Druck, etwa in Form einer aufblasbaren Manschette, von welcher dann unmittelbar, ein aktuell aufgebrachter Druck erfasst werden kann.

Im Rahmen der Erfindung liegt es außerdem, auf anderem Wege als über die Atmung (oder zusätzlich) den venösen Druck zu modulieren, wobei dies bei einem Ausführungsbeispiel etwa dadurch geschehen kann, dass das Ausatmen des Patienten gegen einen Widerstand selbst moduliert wird, etwa mittels einer typischerweise im Bereich zwischen 5 und 50 Hz schwingenden Membran.

## Patentansprüche

1. Vorrichtung zum Bestimmen eines Eich- oder Absolutwerts eines intrakraniellen Druckes (ICP) eines Menschen mit Mitteln (18, 20) zum Erzeugen und/oder Beeinflussen eines vorbestimmten venösen Innendrucks (Pv) im Schädel (10) des Menschen
und einer Sensoreinheit (12), die zur Feststellung des Öffnungszustandes einer Brückenvene (38) im Schädel (10) des Menschen ausgebildet ist, wobei
die Sensoreinheit (12) als Vorrichtung zur intrakraniellen Druckmessung an einem Schädel eines Lebewesens ausgebildet ist, mit zum luftdichten Verschließen eines Gehörganges in die Ohröffnung einsetzbaren Verschlussmitteln (110),
einem den Verschlussmitteln zugeordneten Druckaufnehmer (114), der zum Erfassen eines im verschlossenen Gehörgang vorliegenden Druckes und zum Erzeugen eines elektrisch auswertbaren Signals als Reaktion darauf ausgebildet ist, und einer dem Druckaufnehmer nachgeschalteten Auswerteeinheit, die zum Auswerten des Signals so ausgebildet ist, dass eine einem intrakraniellen Druck und dessen zeitlichem Verlauf entsprechende Information über eine Anzeigeeinheit ablesbar und/oder von einer Ausgabeeinheit so ausgebbar ist, dass Feststellungen über einen intrakraniellen Druck sowie dessen Entwicklung über einen vorbestimmten Zeitraum durch eine Bedienperson möglich sind, wobei
der Vorrichtung zur intrakraniellen Druckmessung eine Öffnungszustands-Detektionseinheit (14) nachgeschaltet ist, die, insbesondere mit Mitteln der digitalen Signalauswertung, als Reaktion auf eine von einer Änderung des venösen Innendrucks im Schädel abhängige, insbesondere sprunghafte, Signaländerung des intrakraniellen Drucksignals den Öffnungszustand der Brückenvene bestimmt.

2. Vorrichtung zum Bestimmen eines Eich- oder Absolutwerts eines intrakraniellen Druckes (ICP) eines Menschen mit Mitteln (18, 20) zum Erzeugen und/oder Beeinflussen eines vorbestimmten venösen Innendrucks (Pv) im Schädel (10) des Menschen und einer Sensoreinheit (12), die zur Feststellung des Öffnungszustandes einer Brückenvene (38) im Schädel (10) des Menschen ausgebildet ist, wobei die Sensoreinheit als Vorrichtung zur intrakraniellen Druckmessung an einem Schädel eines Lebewesens ausgebildet ist, mit einer auf den Schädel abnehmbar aufsetzbaren Vorrichtung (C1), die zum Erfassen einer hirndruckbedingten Relativbewegung zweier Schädelplatten und zum Erzeugen eines elektrisch auswertbaren Bewegungssignals als Reaktion darauf ausgebildet ist, und einer der aufsetzbaren Vorrichtung nachgeschalteten Auswerteeinheit, die zum Auswerten des Signals so ausgebildet ist, dass eine einem intrakraniellen Druck und dessen zeitlichem Verlauf entsprechende Information über eine Anzeigeeinheit ablesbar und/oder von einer Ausgabeeinheit so ausgebbar ist, dass Feststellungen über einen intrakraniellen Druck sowie dessen Entwicklung über einen vorbestimmten Zeitraum möglich sind, wobei der Vorrichtung zur intrakraniellen Druckmessung eine Öffnungszustands-Detektionseinheit (14) nachgeschaltet ist, die, insbesondere mit Mitteln der digitalen Signalauswertung, als Reaktion auf eine von einer Änderung des venösen Innendrucks im Schädel abhängige, insbesondere sprunghafte, Signaländerung des intrakraniellen Drucksignals den Öffnungszustand der Brückenvene bestimmt.

3. Vorrichtung nach Anspruch 1 oder 2, **dadurch gekennzeichnet, dass** die Mittel zum Erzeugen und/oder Beeinflussen des venösen Innendrucks Mittel zum Erzeugen eines positiven endexpiratorischen Druckes (PEP) aufweisen, insbesondere in Form einer einen Gegendruck und/oder Strömungswiderstand für ausgeatmete bzw, in die Mittel eingeblasene Atemluft anbietenden, bevorzugt einstellbaren und/oder geeichten Atemwiderstandsvorrichtung mit einem Mundstück.

4. Vorrichtung nach einem der Ansprüche 1 bis 3, **dadurch gekennzeichnet, dass** die Mittel zur Erzeugung und/oder Beeinflussung des venösen Innendrucks Mittel zum Erzeugen einer definierten Jugulariskompression, insbesondere in Form einer bevorzugt aufblasbaren Manschette; weiter bevorzugt Blutdruckmanschette, aufweisen.

5. Vorrichtung nach einem der Ansprüche 1 bis 4, **gekennzeichnet durch** eine elektronische Steuereinheit (26), die zum Erfassen des dem Öffnungszustand der Brückenvene entsprechenden venösen Innendrucks ausgebildet und zum Herstellen eines elektronischen Rückkopplungskreises mit der Sensoreinheit zu deren Eichung ausgebildet ist.

6. Vorrichtung nach Anspruch 3, **gekennzeichnet durch** Mittel zum Modulieren des PEP, insbesondere mit einem periodischen Wechselsignal einer Frequenz im Bereich zwischen 5 und 50 Hz.

7. Vorrichtung nach Anspruch 6, **dadurch gekennzeichnet, dass** die Mittel zum Modulieren in einen ausgeatmeten Luftstrom geschaltete Schwingungsmittel aufweisen, insbesondere realisiert durch eine mit dem Wechselsignal betriebene Lautsprechermembran.

## Claims

1. Device for determining a calibration or absolute value of an intracranial pressure (ICP) in a human using means (18, 20) for generating and/or influencing a predetermined venous internal pressure (Pv) in the skull (10) of the person and a sensor unit (12) constructed for establishing the state of openness of a bridging vein (38) in the skull (10) of the person, wherein the sensor unit (12) is constructed as a device for intracranial pressure measurement on a skull of a living being having sealing means (110) insertable into the ear orifice for the airtight sealing of an auditory canal, a pressure transducer (114) associated with the sealing means which is constructed for measuring a pressure present in the sealed auditory canal and for generating an electrically evaluable signal in response thereto and an analysing unit connected to the output of the pressure transducer in such a way that an item of information corresponding to an intracranial pressure and its variation over time is readable via a display unit and/or is outputtable by an output unit in such a way that conclusions about an intracranial pressure and its development over a predetermined period of time by an operator are possible, wherein connected to the output of the device for intracranial pressure measurement is a state of openness detection unit (14) which in particular using means of digital signal analysis determines the state of openness of the bridging veins in response to a signal change in the intracranial pressure signal, in particular an abrupt one, dependent on a change in the venous internal pressure in the skull.

2. Device for determining a calibration or absolute value of an intracranial pressure (ICP) in a human using means (18, 20) for generating and/or influencing a predetermined venous internal pressure (Pv) in the skull (10) of the person and a sensor unit (12) constructed for establishing the state of openness of a bridging vein (38) in the skull (10) of the person, wherein the sensor unit is constructed as a device for intracranial pressure measurement on a skull of a living being having a device (C1) removably attachable to the skull which is constructed for detecting a relative movement of two cranial plates caused by brain pressure and for generating an electrically evaluable movement signal in response thereto and an analysing unit connected to the output of the attachable device which is constructed for evaluating the signal in such a way that an item of information corresponding to an intracranial pressure and its variation over time is readable via a display unit and/or is outputtable by an output unit in such a way that conclusions about an intracranial pressure and its development over a predetermined period of time are possible, wherein connected to the output of the device for intracranial pressure measurement is a state of openness detection unit (14) which in particular using means of digital signal analysis determines the state of openness of the bridging veins in response to a signal change in the intracranial pressure signal, in particular an abrupt one, dependent on a change in the venous internal pressure in the skull.

3. Device according to claim 1 or 2, **characterised in that** the means for generating and/or influencing the venous internal pressure comprise means for generating a positive end-expiration pressure (PEP), in particular in the form of a preferably adjustable and/or calibrated respiratory resistance device with a mouthpiece affording a counter-pressure and/or flow resistance to exhaled breath or breath blown into the means.

4. Device according to any of claims 1 to 3, **characterised in that** the means for generating and/or influencing the venous internal pressure comprise means for generating a defined jugular compression, in particular in the form of a preferably inflatable cuff, more preferably blood pressure cuff.

5. Device according to any of claims 1 to 4, **characterised by** an electronic control unit (16) which is constructed for capturing the venous internal pressure corresponding to the state of openness of the bridging veins and for producing an electronic feedback circuit with the sensor unit for the calibration thereof.

6. Device according to claim 3, **characterised by** means for modulating the PEP, in particular using a periodic alternating signal of a frequency in the range between 5 and 50 Hz.

7. Device according to claim 6, **characterised in that** the means for modulating comprise oscillatory means linked into an exhaled stream of air, in particular implemented by a loudspeaker membrane operated by the alternating signal.

## Revendications

1. Dispositif permettant de déterminer une valeur étalon ou absolue d'une pression intracrânienne (ICP) chez l'homme,
- comportant des moyens (18, 20) permettant de générer et/ou d'influencer une pression veineuse interne prédéterminée (Pv) dans le crâne (10) du patient, et comportant une unité de détection (12) permettant de déterminer l'état d'ouverture d'une veine pontante (38) dans le crâne (10) du patient,
dans lequel
• l'unité de détection (12) est conçue sous la forme d'un dispositif de mesure de la pression intracrânienne au niveau du crâne d'un être vivant
• des moyens de fermeture (110) sont conçus de manière à s'insérer dans l'ouverture de l'oreille, pour fermer un conduit auditif de manière étanche à l'air,
• un manomètre (114) associé aux moyens de fermeture conçu de manière à capter une pression régnant dans le conduit auditif fermé et à produire, en réaction à celle-ci, un signal électriquement évaluable, et
• une unité d'évaluation intervenant en aval du manomètre, qui est conçue pour évaluer le signal de manière qu'une information correspondant à une pression intracrânienne et à son évolution temporelle puisse être lue sur une unité d'affichage et/ou émise par une unité de sortie pour permettre à un opérateur de déterminer une pression intracrânienne ainsi que son évolution sur une période donnée,
dans lequel en aval du dispositif de mesure de la pression intracrânienne est disposée une unité de détection de l'état d'ouverture (14) qui détermine l'état d'ouverture de la veine pontante notamment grâce à l'évaluation numérique de signaux, en réaction à une variation du signal de la pression intracrânienne, notamment à une variation brusque du signal dépendant d'une variation de la pression veineuse interne dans le crâne.

2. Dispositif permettant de déterminer une valeur étalon ou absolue d'une pression intracrânienne (ICP) chez l'homme,
- comportant des moyens (18, 20) permettant de générer et/ou d'influencer une pression veineuse interne prédéterminée (Pv) dans le crâne (10) du patient, et comportant une unité de détection (12) permettant de déterminer l'état d'ouverture d'une veine pontante (38) dans le crâne (10) du patient,
dans lequel
• l'unité de détection (12) est conçue sous la forme d'un dispositif de mesure de la pression intracrânienne au niveau du crâne d'un être vivant
• un dispositif (C1) est conçu pour être placé de manière amovible sur le crâne et pour relever un mouvement relatif entre deux plaques crâniennes, conditionné par la pression du cerveau, et pour produire, en réaction à celui-ci, un signal de mouvement électriquement évaluable, et
• une unité d'évaluation intervenant en aval du dispositif amovible, qui est conçue pour évaluer le signal de manière qu'une information correspondant à une pression intracrânienne et à son évolution temporelle puisse être lue sur une unité d'affichage et/ou émise par une unité de sortie pour permettre de déterminer une pression intracrânienne ainsi que son évolution sur une période donnée,
dans lequel en aval du dispositif de mesure de la pression intracrânienne est disposée une unité de détection de l'état d'ouverture (14) qui détermine l'état d'ouverture de la veine pontante notamment grâce à l'évaluation numérique de signaux, en réaction à une variation du signal de la pression intracrânienne, notamment à une variation brusque du signal dépendant d'une variation de la pression veineuse interne dans le crâne.

3. Dispositif selon la revendication 1 ou 2, **caractérisé en ce que** les moyens permettant de générer et/ou d'influencer la pression veineuse interne présentent des moyens de production de pression positive expiratoire (PEP), sous la forme notamment d'un dispositif de résistance respiratoire à embout buccal qui est de préférence réglable et/ou étalonné et offre une contre-pression et/ou une résistance pour l'air respiratoire expiré ou insufflé dans les moyens.

4. Dispositif selon l'une des revendications 1 à 3, **caractérisé en ce que** les moyens permettant de générer et/ou d'influencer la pression veineuse interne présentent des moyens de production d'une certaine compression jugulaire, sous la forme notamment d'un brassard de préférence gonflable et plus préférablement d'un brassard à tension sanguine.

5. Dispositif selon l'une des revendications 1 à 4, **caractérisé par** une unité de commande électronique (16) conçue pour relever la pression veineuse interne correspondant à l'état d'ouverture de la veine pontante et pour produire un circuit de rétrocouplage électronique avec l'unité de détection à des fins d'étalonnage.

6. Dispositif selon la revendication 3, **caractérisé par** des moyens de modulation de la PEP, notamment au moyen d'un signal alternatif périodique d'une fréquence située dans la plage de 5 à 50 Hz.

7. Dispositif selon la revendication 6, **caractérisé en ce que** les moyens de modulation présentent des moyens vibrants placés dans un courant d'air expiré, réalisés notamment par une membrane de haut-parleur activée par le signal alternatif.
